# EUROPEAN PATENT SPECIFICATION

(11) **EP 2 575 622 B1**
(45) Date of publication and mention of the grant of the patent: **27.04.2016**
(21) Application number: 10731562.4
(22) Date of filing: 03.06.2010
(51) Int. Cl.: G01S 7/52, G01S 15/89, G09G 5/08, G06F 3/0346, A61B 8/00

(54) **CONTROL DEVICE**
STEUERVORRICHTUNG
DISPOSITIF DE COMMANDE

(43) Date of publication of application: 10.04.2013
(73) Proprietor: B-K Medical ApS, 2730 Herlev (DK)
(72) Inventor: JENSEN, Ole, C., DK-3500 Vaerloese (DK)
(74) Representative: Quintelier, Claude
(86) International application number: PCT/IB2010/001348
(87) International publication number: WO 2011/151664

(56) References cited:
- WO-A2-2008/146201
- JP-A- 2007 222 322
- US-A1- 2007 016 025
- US-A1- 2007 066 894
- US-A1- 2007 078 340
- US-A1- 2009 217 207
- US-B1- 6 290 649

## Description

### TECHNICAL FIELD

The following generally relates to a control device and is described herein with particular application to an ultrasound imaging system; however, the control device can be used in connection with other applications.

### BACKGROUND

Ultrasound scanners provide useful information about the interior characteristics of an object under examination. In medical applications, clinicians have used ultrasound scanners to examine human subjects in settings such as hospitals, physician's offices, and other locations. Ultrasound scanners have been used in the emergency room, operating room, and similar environments. Such environments, at times, are sterile environments. For example, an operating room during an invasive surgical procedure is kept a sterile environment. In such an environment, the surgeon may utilize an ultrasound scanner at some point during the surgery to locate, measure a size of, and/or determine other characteristics about tissue of interest such as a tumor or suspected tumor.

By way of example, the surgeon places the transducer on the body over the tissue of interest and begins scanning. A display displays the information sensed by the transducer. The surgeon may desire to change scanner parameters or settings, or determine a characteristic about the displayed information. For example, the surgeon may want to increase the gain, adjust depth, change the zoom factor, switch scanning modes (e.g., B-mode to Doppler), measure a size of a suspected tumor, observe blood flow through the tissue, etc. Such actions have required user interaction with the ultrasound console. For example, the positioning and/or movement of a cursor on the display and/or a selection of an action through the display generally have required use of a keyboard/keypad and/or a mouse/trackball/touchpad.

One approach to interacting with the ultrasound system is to have another person in the operating room control the system through the above noted input devices as the surgeon provides them with verbal instructions. However, this requires trained/instructed personnel and tends to be slow as the instruction will often be in steps and in one direction at a time (e.g., up, up, up, left, left, left...). Another approach is to have the surgeon use a device such as a trackball, mouse, touchpad, or the like covered with or without a sterile cover. Unfortunately, the frictional resistance and/or the stickiness between the finger and the device and/or cover can substantialy vary depending on fluids and/or material such as wet gel, blood, etc. on the finger and/or device or cover, which may render suitable positioning of the cursor a difficult and time consuming task. Another approach is to have the surgeon use a joystick or direciton keys. However, movement therewith generally is indirect (i.e., sets the direcion and speed) and thus can be slower and relatively less precise.

US-A-2007/016025 describes a post-imaging system for medical diagnostic ultrasound imaging which provides three-dimensional navigation. A volume object is selected, for example, a cube or organ-shaped object, from a three-dimensional rendering of an ultrasound image acquired using one or more scanning devices. As the volume object is rotated, that is, the viewing angle is changed by a user, the viewing direction associated with the three dimensional rendering also rotates. A sensor is provided for sensing an outer surface of the volume object and determines an orientation and/or position in three-dimensions thereof so that the viewing angle can correctly be displayed.

US-B-6290649 describes a system in which a transducer probe is moved adjacent an object to be imaged or in free space. Three-dimensional movement, including roll, pitch and displacement, of the transducer probe is sensed by a position sensor which generates position data for transmitting to a host computer. The signals are stored in a memory and shown on a display. A selection signal, for example, resulting from the operation of a switch, as well as the position data obtained from a sensor are used to manipulate image data corresponding to cut planes through a three-dimensional volume in which the object is located. A cursor is provided on the display which is activated by movement of the transducer probe and operation of the switch.

US-A-2009/0217207 described a device for displaying a three-dimensional medical image on a display element connected to a processing unit. Raw data is measured by a medical imaging system and processed by the processing unit to generate a three-dimensional medical image for display. The image can be changed by moving cursor keys on a computer keyboard or scrolling using a computer mouse. A remote control is associated with the device and includes a three-axis acceleration sensor and a three-axis rotary rate sensor. Any movement of the remote control can be detected by the sensors and positional information is generated therefrom by the processing unit 13 in accordance with display criteria set by one or more control selection switches.

JP-A-2007/222322 describes a transceiver unit for ultrasound scanning of a target organ of a subject, an image of the target organ is generated by an image generating device and displayed. A control device is provided for remotely controlling the transceiver unit and associated apparatus. Positional information of a scanning device is determined to provide three-dimensional control over display of the image.

US-A-2007/0078340 discloses an ultrasound imaging system in which an imaging transducer connected thereto is used to provide control signals. Specific patterns of motion of the transducer made by an operator of the system are detected by the system and converted into control signals if the patterns of motion match known pattern templates. Accelerometers are used to determine motion in x-, y- and z-directions made by the operator and a rate gyro is used to sense twisting or rolling motion of the wrist of the operator.

### SUMMARY

Aspects of the application address the above matters, and others.

In accordance with one aspect of the invention, an imaging system is provided which comprises an ultrasound imaging apparatus having a scanner which includes an interface; an ultrasound transducer probe including an interface connectable to the interface of the scanner, the interfaces including suitable pathways and/or connectors for conveying control and/or data signals between the ultrasonic transducer probe and the scanner, the ultrasound transducer probe comprising one of: a single element transducer and a multiple element transducer array; and a display including a graphical cursor associated therewith; characterized in that the imaging system further comprises: a control device including a position sensing component that senses a position of the control device relative to a free space coordinate system and generates a signal indicative thereof, and a transmitter that transmits the signal to the imaging system that employs the signal to position the graphical cursor on the display of the imaging system in coordination with the position of the position sensing component in free space, the position of the graphical cursor at least being operable for selecting a scanning mode of the scanner through an on-screen menu; and in that the control device further comprises at least one user control that controls the imaging system, the at least one user control being operable for at least remotely changing the scanning mode.

In accordance with another aspect of the present invention, there is provided a method of operating an imaging system comprising a scanner; an ultrasound transducer probe; a display; and a control device, the control device including a position sensing component operable for sending a position thereof relative to a free space coordinate system and for generating a signal indicative thereof, and a transmitter operable for transmitting the signal to the scanner,, the method comprising the steps of: a) receiving, via the scanner, a request to register the control device therewith; b) transmitting, by the scanner, a notification indicating that the control device is registered therewith; c) receiving, at the scanner, the signal indicative of a position in free space of the registered control device; d) positioning, by the scanner, the graphical cursor of the display of the scanner based on the position of the registered control device in free space; e) selecting a first scanning mode of the scanner via an on-screen menu; and f)receiving, at the scanner, a second signal that changes scanning mode thereof.

In one aspect, a control device includes a position sensing component that senses a position of the wireless control device relative to a free space coordinate system and generates a signal indicative thereof. The control device also includes a transmitter that transmits the signal to an imaging system that employs the signal to position a graphical cursor on a display of the imaging system in coordination with the position of the sensing component in free space.

In another aspect, an imaging apparatus includes a scanner with an interface configured to connect with a complementary interface of an imaging transducer probe. The scanner also includes a display that presents information indicative of a signal sensed by the imaging transducer probe. The scanner also includes a processor that generates and positions a graphical cursor of a display region of the display based on a position of a control device, which is registered to operate the scanner, in free space.

In another aspect, an imaging system includes a control device and an imaging apparatus. The control device includes a position sensing component that senses a position of the control device in free space and generates a signal indicative thereof and a transmitter that transmits the signal. The imaging apparatus includes a display, a receiver that receives the signal transmitted by the control device, and a processor that generates, displays, and positions a graphical cursor on the display based on the received signal.

In another aspect, a method includes transmitting, via a control device, a request to register the control device with a scanner. The method further includes receiving, by the control device, a notification indicating that the control device is registered with the scanner. The method further includes transmitting, via the control device, a signal indicative of a position in free space of the registered control device, wherein the signal is received by the scanner, which positions a cursor on a display of the scanner based on the location of the registered control device in free space.

In another aspect, a method includes receiving, via a scanner, a request to register a control device with the scanner. The method further includes transmitting, by the scanner, a notification indicating that the control device is registered with the scanner. The method further includes receiving, at the scanner, a signal indicative of a position in free space of the registered control device. The method further includes positioning, by the scanner, a cursor of a display of the scanner based on the position of the registered control device in free space.

Those skilled in the art will recognize still other aspects of the present application upon reading and understanding the attached description.

### BRIEF DESCRIPTION OF THE DRAWINGS

The application is illustrated by way of example and not limitation in the figures of the accompanying drawings, in which like references indicate similar elements and in which:
FIGURE 1 illustrates an example imaging system utilizing a control device to operate a scanner thereof;
FIGURE 2 illustrates an example control device and scanner;
FIGURE 3 illustrates an example implementation of the control device;
FIGURE 4 illustrates an example method for employing the control device to operate the scanner; and
FIGURE 5 illustrates an example method for operating the scanner using the control device.

### DETAILED DESCRIPTION

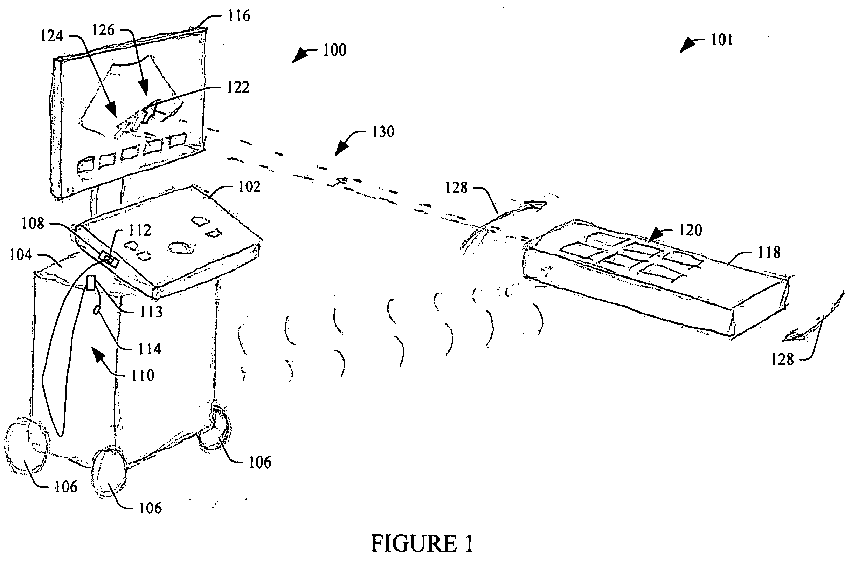

Figure 1 illustrates an imaging system 101. The imaging system 101 includes an imaging apparatus 100 such as an ultrasound imaging apparatus. The imaging apparatus 100 includes a scanner 102 affixed or mounted to a base 104. The illustrated base 104 includes wheels 106, which allow the scanner 102 to be rolled from location to location such as operating room to operating room or other location. In another embodiment, the scanner 102 is removeably attached to the base 104. In such an embodiment, the scanner 102 may be portable in that it can be removed from the base 104 and carried, in one or two hands or otherwise by the operator, and attached to another base or employed without a base. In yet another embodiment, the base 104 may not include wheels. In this embodiment, the base 104 may be mechanically attached to a surface such as a floor, ceiling, wall, table, etc., or free standing on the surface.

The scanner 102 includes an interface 108 configured to connect with a complementary interface of an ultrasound transducer probe. In the illustrated embodiment, an ultrasound transducer probe 110 includes a complementary interface 112 that is connected to the interface 108 of the scanner 102. The interfaces 108 and 112 include suitable pathways and/or connectors for conveying control and/or data signals between the probe 110 and the scanner 102. The probe 110 used with the scanner 102 may include a single element transducer or a multiple element transducer array, which captures information for two dimensional, three dimensional, four dimensional, Doppler, and/or other ultrasounds imaging modes. The illustrated probe 110 is shown being supported by a probe holder 113 of the scanner 102. The interface 108 may be configured to alternately interface with other probes, for example, different frequency probes, probes with different capabilities, and/or other probes.

The scanner 102 also includes an input device such as a keyboard, a touchpad, and the like. The keyboard and/or the touchpad can be used to navigate through the displayed application menus, set parameters, select actions, etc. The apparatus 100 also includes a human readable display such as a monitor 116. The monitor 116 may be an LCD, plasma, CRT, or other monitor. Information captured by the transducer 114 is processed by one or more processors of the scanner 102 and presented or displayed via the monitor 116. Also displayed on the monitor 116 are application menus, soft controls, a cursor, and the like which can be used for setting parameters, making measurements, controlling operation, etc. Software resident on the scanner 102 allows the operator to control the operation of the apparatus 100, for example, by allowing the operator to select a scanning mode, initiate scanning, measure characteristics of scanned features, etc.

A control device 118 is configured to register with or pair with the scanner 102. In the illustrated embodiment, the control device 118 is wireless. However, embodiments in which the control device 118 is configured as a wired device are also contemplated herein. The wireless control device 118 is configured to remotely control at least one operation of the scanner 102 based on a user input and/or action. As described in greater detail below, in one embodiment, the wireless control device 118 transmits position information (e.g., magnitude and/or direction, location, etc.) about the wireless control device 118 in free space to the scanner 102, which uses such information, for example, to move a cursor displayed on the monitor 116 and/or track the cursor to the movement of the wireless control device 118. This is illustrated in FIGURE 1. As shown, a cursor 122 is moved from a first position 124 to a second position 126 in response to moving the wireless control device 118 as indicated by the arrows 128. Dashed lines 130 are provided merely to show an example mapping between movement of the wireless control device 118 and the position of the cursor 122. The illustrated wireless control device 118 includes one or more user controls 120 such as buttons or the like which can be used to remotely change the gain, depth, zoom, scanning mode, etc., measure a size of a suspected tumor, observe blood flow through the tissue, etc.

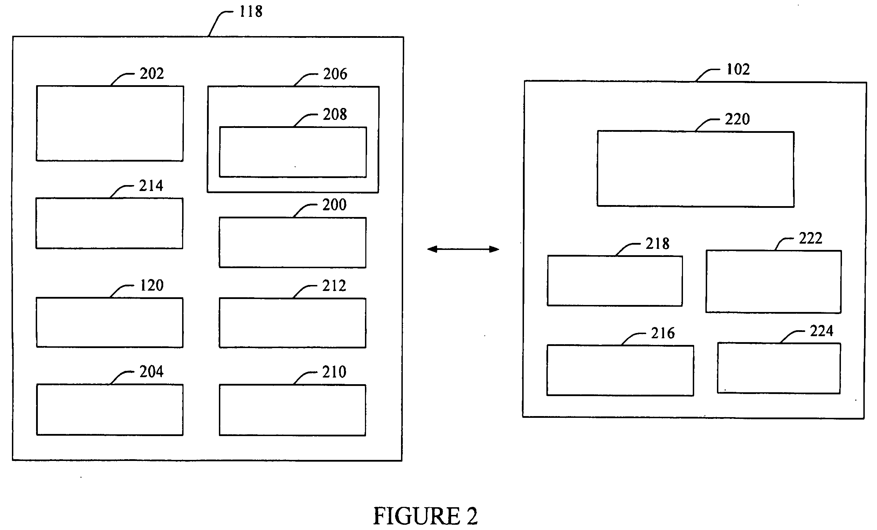

Figure 2 illustrates a non-limiting example of the wireless control device 118 and the scanner 102 paired together for communication therebetween.

The illustrated wireless control device 118 includes a position sensing component 202 that senses a position of the position sensing component 202 in free space, and hence the wireless control device 118 in free space. In one embodiment, the position sensing component 202 includes a single and/or multi-axis accelerometer, which can be configured to sense magnitude and/or direction in relation to acceleration, motion, vibration, etc. of the wireless control device 118, and generates a signal indicative thereof. A suitable accelerometer may be part of a micro electro-mechanical systems (MEMS) or other circuitry. Additionally or alternatively, the position sensing component 202 includes a gyroscope, which is configured to sense an orientation of the sensing component 202 and generate a signal indicative thereof. The position sensing component 202 may additionally or alternatively includes one or more other devices such as magnetic, light, video tracking, etc. based devices that can sense absolute and/or relative position, movement, motion, and the like.

Memory 206 stores various information, including, but not limited to data, such as the signal generated by the position sensing component 202, a unique identification 208 (if configured with one), control instructions, and/or other information.

User controls 120 can be used to initiate communication with, communicate with and cease communication with the scanner 102. By way of non-limiting example, in one embodiment in which only a registered wireless control device can communicate with the scanner 102, the user controls 120 are used to send a registration request to the scanner 102. As discussed in greater detail below, registration can be achieved based on the unique identification 208 in the memory 206. In another embodiment, the user controls 120 can be used to set/change the scanning mode (e.g., B-mode, Doppler, color, etc.), increase gain, adjust depth, zoom in or zoom out, activate and employ a measurement tool, invoke a menu to be displayed, capture an image, freeze an image, and/or other features when registered with the scanner 102. The user controls 120 are also used to turn the wireless control device 1118 on and off. The same or a different control (or other mechanism) can be used to terminate and/or change registration.

A power source 204 includes a primary (non-rechargeable) battery, a secondary (rechargeable) battery, a super capacitor, and/or the like that supplies power for one or more of the components of the wireless control device 118. In another embodiment, the wireless control device 118 includes an internal charger or an interface to an external charger for charging a secondary battery thereof. In another embodiment, the wireless control device 118 includes a power converter for converting power from an outside source such as a power cube, a power supply, an AC receptacle, or other power source.

One or more microprocessor 214 facilitates operation of the wireless control device 118. The one or more microprocessor 214 may also be configured to determine various information, which can be stored in the memory 206, conveyed to the scanner 102, presented to the user (e.g., via an audible, visual, or tactile manner), etc. For example, where the power source 204 includes a consumable power source such as a battery, the one or more microprocessor 214 may determine a state of the battery, such as a charge current state, an expected end of life state, and/or other state. This information can be used to inform the scanner 102 and/or a user about the state of the power source 204.

A transmitter 210 is configured to transmit data and/or control signals to scanner 102. For example, the transmitter 210 can transmit the signal generated by the position sensing component 202, the unique identification 208, power source power state information, and/or other information. A receiver 212 is configured to receive information at least from the scanner 102. In another embodiment, the receiver is omitted.

One or more indicators 200 such as a display screen, a visual indicator such as a light emitting diode (LED), an audible indicator, a tactile indicator, etc. provide feedback to an operator. Suitable feedback may relate to the registration process and indicate successful and unsuccessful registration. The suitable may also provide information about the power source such as an indication that a replaceable power source thereof need to be replaced or that another wireless control device 118 should be obtained based on the state of the power source.

The scanner 102 includes receiver 216, which is configured to receive communications from the wireless control device 118 and/or other devices such as one or more other wireless control devices. Suitable received information includes, but is not limited to, the signal generated by the position sensing component 202, the unique identification 208, information related to the state of the power source, a control signal, and/or other information. A transmitter 218 is configured to transmit information to one or more wireless control device 118 and/or other devices. Such information may include notifications indicative of a registration state between the wireless control device 118 and the scanner 102. In another embodiment, the transmitter 218 is omitted.

A wireless control device registration component 220 pairs or registers the scanner 102 with a particular wireless control device 118 for communication therewith. For example, the wireless control device registration component 220 may receive the unique identification 208 and subsequently register the wireless control device 118 based on the unique identification. In one embodiment, only a registered wireless control device can control the scanner 102 and/or respond to the scanner 102. As such, the scanner 102 can differentiate between communication provided by a wireless control device 118 registered to be used with the scanner 102 and a wireless control device not registered to be used with the scanner 102, for example a wireless control device 118 in a neighboring operating room that is configured for communication with a different scanner 102.

The wireless control device registration component 220 can also be used to terminate registration and/or change registration, for example, in connection with another scanner 102. In another embodiment, multiple wireless control devices 118 can be concurrently registered with the scanner 102. This may allow for use of multiple wireless control devices 102 in the same operating room, for example, where multiple personnel may be authorized to control the scanner 102, where multiple wireless control devices 118 are placed in different location in the operating room, for example, for convenience of the surgeon, etc.

A coordinate space mapper 222 maps the coordinate space of the relative position of the wireless control device 118 (e.g., as determined from the signal from the position sensing component 202) to the display coordinate space of the display 116. A processor 224 uses this mapping to map the wireless control device 118 to the cursor and position the cursor within the display window of the display based on the free hand movement of the wireless control device 118. The mapping can also be used to map other displayed items based on the position of the wireless control device 118. The processor 224 executes computer readable medium embed or encoded in computer readable medium to perform various operations such as generating, displaying, and positioning the cursor on the display region of the display.

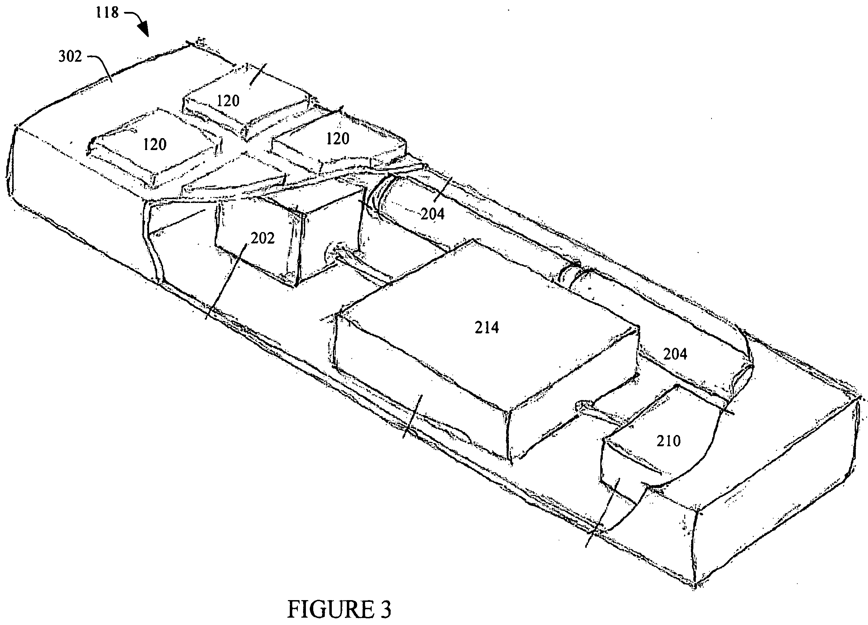

Figure 3 illustrates an example wireless control device 118. The wireless control device 118 is shown with a section cut out, revealing the components therein. In this example, the wireless control device 118 is packaged so that it can be carried and operated using a signal hand.

An outside shell 302 may provide a hermetically sealed device, which may mitigate fluids from entering the wireless control device 118. In another embodiment, the shell may not provide hermetically sealed device. The outside shell 302 may also include a material(s) which allows the wireless control device 118 to be disinfected and/or sterilized through temperature, chemical, optical, radiation, and/or other approaches.

With the illustrated embodiment, the power source 204 includes a set of batteries. Where the wireless control device 118 is configured to be disposable and cannot be opened, the wireless control device 118 is discarded after one or more uses or once the power source 204 drains to a predetermined level. Where the batteries are replaceable, the shell 320 includes a removeable section, providing access to a battery receiving region, and the batteries can be replaced after they are drained to a predetermined charge level.

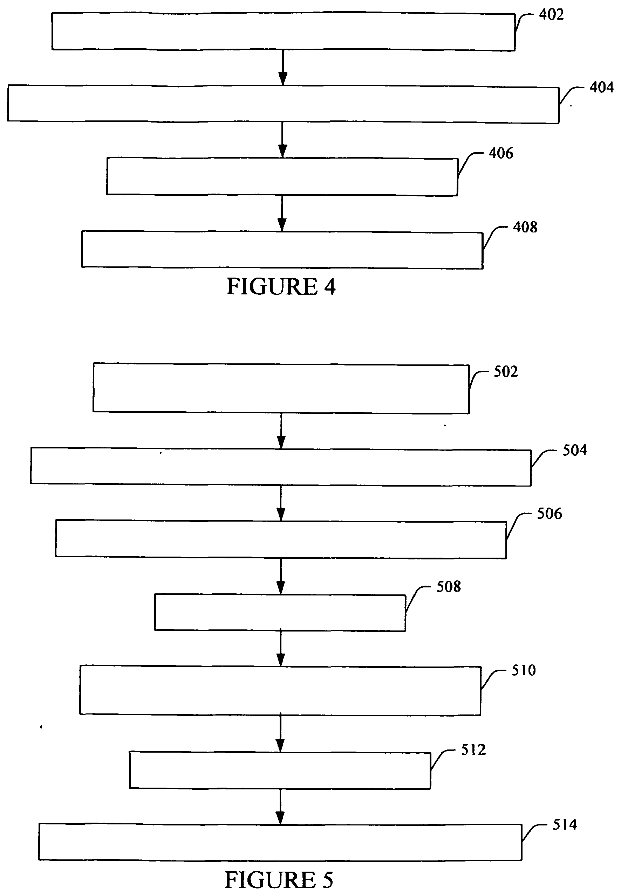

FIGURE 4 illustrates an example method for employing the wireless control device 118 to operate the scanner 102.

At 402, the wireless control device 118 transmits a registration request to a scanner 102, for example, in response to a user input via the user controls 120. The registration request may include or is sent along with the unique identification 208 of the wireless control device 118.

At 404, the wireless control device 118 receives a signal indicative of a registration with the scanner 102. The wireless control device 118 may provide a visual, audible, tactile, and/or other notification, indicating the registration.

At 406, in response to a user input via the user controls 120, the wireless control device 118 transmits a control signal to the scanner 102. The control signal may be associated with the scanning mode (e.g., B-mode, Doppler, color mode, etc.), initiating, pausing and terminating scanning, setting a viewing parameter (e.g., gain, depth, zoom, etc.), invoking a measurement application, etc.

At 408, at least where the control signal invokes display of the cursor 122 on the display 116, the wireless control device 118 continuously or periodically transmits a signal indicative of its location in 3D free space. The signal is employed by the scanner to position and/or update the position of the cursor 122 on the display 116 in accordance with the location of the wireless control device 118 in 3D free space. The cursor can be used to identify and/or select one or more region, for example, to make measurement, determine characteristics, etc.

FIGURE 5 illustrates an example method for operating the scanner 102 using the wireless control device 118.

At 502, the scanner 102 receives a registration request to registers the scanner 102 with a wireless control device 118.

At 504, the scanner 102 registers the wireless control device 118 for use with the scanner 102. The use may be one to one such that only the registered wireless control device 118 can communicate and/or control the scanner 102.

At 506, the scanner 102 transmits a signal indicative of the registration.

At 508, the scanner 102 receives a control signal from the wireless control device 118. The control signal may be associated with the scanning mode (e.g., B-mode, Doppler, color mode, etc.), a viewing parameter (e.g., gain, depth, zoom, etc.), etc., invoking a measurement application, etc.

At 510, at least where the control signal invokes display of the cursor 122 on the display 116, the scanner 102 receives a signal indicative of a position of the wireless control device 118 in 3D free space.

At 512, the scanner 102 maps the signal to the display space of the monitor 116.

At 514, the scanner 102 displays the cursor 122 on the display at a position that corresponds to the location of the wireless control device 118 in 3D free space. This includes moving the cursor 122 in coordination with movement of the wireless control device 118.

The following provides an example use-case scenario for using the wireless control device 118 with an ultrasound imaging system in a surgical setting.

A patient and the operating room are prepared for the surgery. A wireless control device 118 is obtained from a warehouse/stocking area. The wireless control device 118 along with one or more other sterile items are placed on a moveable cart or rolling table. The wireless control 118 and the other items are unpacked and laid out on a sterile cloth or the like, which covers the table. Where the package is not sterile, non-sterile personnel can open the package, and sterile personnel can remove the items and place them on the sterile cloth. Where the package is sterile, sterile personnel can both remove and place the items n the cloth.

The scanner, if not already in the operating room, is brought into the operating room. Generally, it is placed opposite the surgeon or on the same side of the surgical table as the surgeon. The scanner is connected and turned on. A transducer is brought in unpacked like the other sterile items and placed on a sterile table close to the scanner. The transducer is prepared and connected to the scanner. The scanner is checked. This may include checking to see if there is an image. The wireless control device 118 is registered with the scanner as described herein or otherwise. Where the wireless control device 118 provides power source information, the scanner may display a message indicating the current state of the power source.

The surgery begins. When the surgeon wants to identify the location of tissue of interest such as a tumor, the surgeon asks for the ultrasound. Sterile personnel hands the transducer and the wireless control device 118 to the surgeon. Alternatively, the surgeon picks up the wireless control device 118 from the sterile cloth. The surgeon may check that the wireless control device 118 works. The surgeon places the wireless control device 118 within reach. The surgeon begins scanning the tissue. If the surgeon wants to increase the gain, the surgeon picks up the wireless control device 118 with a free hand and uses the wireless control device 118 to adjust the gain.

After finding the tumor, the surgeon may want to check so see if there are any vessels that should be avoided when cutting. To accomplish this, the surgeon may use the wireless control device 118 to change the scanner mode to Color mode, for example, by pushing a dedicated user control 120 on the wireless control device 118 or selecting the mode via the cursor 122 and an on-screen menu. The surgeon can use the wireless control device 118 to adjust the color gain, again, via dedicated control 120 or cursor 122 and an on-screen menu.

The surgeon can also measure the tumor (e.g., the diameter thereof) using the wireless control device 118. For this, the surgeon activates measurement mode via dedicated user control 120 or on-screen menu. The surgeon then positions the cursor 122 via moving the wireless control device 118 and uses a user control 120 to select a position. With the same cursor 122 or a different cursor 122, the surgeon selects a second position. The scanner 102 determines a distance between the two points and displays the distance on the monitor 116. One or more such measurements or other measurements (e.g., perimeter, area, etc.) can be obtained.

After the surgeon is finished using the scanner 102, the wireless control device 118 and the scanner 102 are unregistered. This may be accomplished through the wireless control device 118 or the scanner 102. The sterile cloth is wrapped around the sterile items, including the wireless control device 118, and these items are taken to the cleaning area. If a sterile bag/cover was used with the wireless control device 118, the bag/cover is removed and disposed. The wireless control device 118 is washed and disinfected, for example, via rubbing alcohol, and brought/sent to sterilization. Once sterilized, the wireless control device 118 is packed away and kept in a sterile location and/or container, for example, back in the warehouse/stocking area, and is ready for subsequent use, as just described or otherwise.

Although the above is described in the context of an imaging apparatus such as an ultrasounds imaging apparatus, it is to he understood the other modality imaging apparatuses and non imaging apparatuses are also contemplated herein. Other suitable applications include application in which a cursor and/or other displayed graphical object can be moved on a display.

The application has been described with reference to various embodiments. Modifications and alterations will occur to others upon reading the application. It is intended that the invention be construed as including all such modifications and alterations, including insofar as they come within the scope of the appended claims and the equivalents thereof.

## Claims

1. An imaging system (101) comprising:
an ultrasound imaging apparatus (100) having a scanner (102) which includes an interface (108);
an ultrasound transducer probe (110) including a complementary interface (112) connectable to the interface (108) of the scanner (102), the interfaces (108, 112) including suitable pathways and/or connectors for conveying control and/or data signals between the ultrasonic transducer probe (110) and the scanner (102), the ultrasound transducer probe (110) comprising one of: a single element transducer and a multiple element transducer array; and
a display (116) including a graphical cursor (122) displayed thereon;
**characterized in that** the imaging system (101) further comprises:
a wireless control device (118) including a position sensing component (202) configured for sensing a position of the wireless control device (118) relative to a free space coordinate system and for generating a signal indicative thereof, and a transmitter (210) configured for transmitting the signal to the imaging system (101), the imaging system (101) being configured to employ the signal to position the graphical cursor (122) on the display (116) in coordination with the position of the position sensing component (202) in free space, the position of the graphical cursor (122) being operable for selecting a scanning mode of the scanner (102) through an on-screen menu;
and **in that** the wireless control device (118) further comprises at least one user control (120) that controls a parameter of the imaging system (101), the parameter being operable for remotely changing the scanning mode.

2. The imaging system of claim 1, wherein the control device (118) further comprises a memory (206) including a unique identification (208), the transmitter (210) transmitting the unique identification (208) to the imaging system (101) for registering the wireless control device (118) in order to operate the imaging system (101) based on the unique identification.

3. The imaging system of claim 2, wherein the wireless control device (118) is configured only to operate the imaging system (101) to which it is registered.

4. The imaging system of any of claims 1 to 3, wherein the wireless control device (118) further comprises:
an indicator (200) that provides at least one of: a visual, audible, or tactile indication in response to a successful registration between the wireless control device (118) and the imaging system (101).

5. The imaging system of any of claims 1 to 4, wherein the parameter also corresponds to at least one of: a gain, a depth, a zoom, and image capture.

6. The imaging system of claim 5, wherein the at least one user control (120) is configured to invoke an application which employs the graphical cursor (122).

7. The imaging system of any of claims 1 to 6, wherein the wireless control device (118) is at least one of disinfectable or sterilizeable.

8. The imaging system of any of claims 1 to 7, wherein the wireless control device (118) is a hand-held portable device.

9. The imaging system of any of claims 1 to 8, wherein the position sensing component (202) includes an accelerometer that determines a magnitude and direction information for the wireless control device (118) in free space and the signal includes the magnitude and direction information.

10. The imaging system of any of claims 1 to 9, wherein the position sensing component (202) includes a gyroscope that determines orientation information for the wireless control device (118) in free space and the signal includes the orientation information.

11. The imaging system of any of claims 1 to 10, wherein the scanning mode of the scanner (102) comprises at least one of: B-mode, Doppler and color.

12. A method of operating an imaging system (101) according to any of the preceding claims, the method comprising the steps of:
a) receiving, via the scanner (102), a request to register the wireless control device (118) therewith;
b) transmitting, by the scanner (102), a notification indicating that the wireless control device (118) is registered therewith;
c) receiving, at the scanner (102), a signal indicative of a position in free space of the registered wireless control device (118);
d) positioning, by the scanner (102), the graphical cursor (122) of the display (116) based on the position of the registered wireless control device (118) in free space; and
e) selecting a scanning mode of the scanner (102) via an on-screen menu.

13. The method of claim 12, further comprising the steps of:
generating a mapping between the position of the registered wireless control device (118) and a coordinate system of the display (116); and
positioning the graphical cursor (122) of the display (116) based on the mapping.

14. A method of operating an imaging system (101) according to any of claims 1 to 11, the method comprising the steps of:
a) receiving, via the scanner (102), a request to register the wireless control device (118) therewith;
b) transmitting, by the scanner (102), a notification indicating that the wireless control device (118) is registered therewith; and
c) receiving, at the scanner (102), a signal from the user control of the control device (118) that remotely changes the scanning mode thereof.

15. The method of any of claims 12 to 14, further comprising the step of:
controlling at least one of: a gain, a depth, image capture, or a zoom of the scanner (102) based on a signal provided by the wireless control device (118).

## Patentansprüche

1. Bildgebungssystem (101) umfassend:
ein Ultraschallbildgebungsgerät (100), das einen Scanner (102) aufweist, welcher eine Schnittstelle (108) enthält;
eine Ultraschallwandlersonde (110) einschließlich einer komplementären Schnittstelle (112), die an die Schnittstelle (108) des Scanners (102) angeschlossen werden kann, wobei die Schnittstellen (108, 112) geeignete Wege und/oder Anschlüsse zum Transportieren von Steuer- und/oder Datensignalen zwischen der Ultraschallwandlersonde (110) und dem Scanner (102) enthalten, wobei die Ultraschallwandlersonde (110) eines der folgenden Elemente umfasst: einen Wandler für ein einzelnes Element und eine Wandleranordnung für mehrere Elemente; und
eine Anzeige (116) einschließlich eines grafischen Cursors (122), der darauf angezeigt wird;
**dadurch gekennzeichnet, dass** das Bildgebungssystem (101) ferner Folgendes umfasst:
eine drahtlose Steuervorrichtung (118) einschließlich einer Positionserfassungskomponente (202), die ausgebildet ist, um eine Position der drahtlosen Steuervorrichtung (118) bezüglich eines Freiraumkoordinatensystems zu erfassen und ein Signal, dass diese anzeigt, zu erzeugen, und einen Transmitter (210), der ausgebildet ist, um das Signal an das Bildgebungssystem (101) zu übertragen, wobei das Bildgebungssystem (101) ausgebildet ist, um das Signal zu verwenden, um den grafischen Cursor (122) auf dem Display (116) im Zusammenspiel mit der Position der Positionserfassungskomponente (202) im Freiraum zu positionieren, wobei die Position des grafischen Cursors (122) zum Auswählen eines Scanmodus des Scanners (102) durch ein Bildschirmmenü bedient werden kann;
und dass die drahtlose Steuervorrichtung (118) ferner mindestens eine Benutzersteuerung (120) umfasst, die einen Parameter des Bildgebungssystems (101) steuert, wobei der Parameter zum Ändern des Scanmodus aus der Ferne bedient werden kann.

2. Bildgebungssystem nach Anspruch 1, wobei die Steuervorrichtung (118) ferner einen Speicher (206) umfasst, der eine einzigartige Kennung (208) enthält, wobei der Transmitter (210) die einzigartige Kennung (208) an das Bildgebungssystem (101) zum Registrieren der drahtlosen Steuervorrichtung (118) überträgt, um das Bildgebungssystem (101) auf Grundlage der einzigartigen Kennung zu bedienen.

3. Bildgebungssystem nach Anspruch 2, wobei die drahtlose Steuervorrichtung (118) ausgebildet ist, um lediglich das Bildgebungssystem (101), für welches sie registriert ist, zu bedienen.

4. Bildgebungssystem nach einem der Ansprüche 1 bis 3, wobei die drahtlose Steuervorrichtung (118) ferner Folgendes umfasst:
ein Anzeigegerät (200), das mindestens eines der folgenden Angaben bereitstellt: eine visuelle, hörbare oder berührbare Angabe als Reaktion auf eine erfolgreiche Registrierung zwischen der drahtlosen Steuervorrichtung (118) und dem Bildgebungssystem (101).

5. Bildgebungssystem nach einem der Ansprüche 1 bis 4, wobei der Parameter auch mindestens einem der Folgenden entspricht: einer Verstärkung, einer Tiefe, einem Zoom und einer Bilderfassung.

6. Bildgebungssystem nach Anspruch 5, wobei die mindestens eine Benutzersteuerung (120) ausgebildet ist, um eine Anwendung aufzurufen, welche den grafischen Cursor (122) verwendet.

7. Bildgebungssystem nach einem der Ansprüche 1 bis 6, wobei die drahtlose Steuervorrichtung (118) mindestens eine einer desinfizierbaren oder sterilisierbaren drahtlosen Steuervorrichtung ist.

8. Bildgebungssystem nach einem der Ansprüche 1 bis 7, wobei die drahtlose Steuervorrichtung (118) eine tragbare Handheld-Vorrichtung ist.

9. Bildgebungssystem nach einem der Ansprüche 1 bis 8, wobei die Positionserfassungskomponente (202) einen Beschleunigungsmesser enthält, der eine Größen- und Richtungsinformation für die drahtlose Steuervorrichtung (118) in einem Freiraum bestimmt, und wobei das Signal die Größen- und Richtungsinformation enthält.

10. Bildgebungssystem nach einem der Ansprüche 1 bis 9, wobei die Positionserfassungskomponente (202) ein Gyroskop enthält, das Ausrichtungsinformationen für die drahtlose Steuervorrichtung (118) im Freiraum bestimmt, und wobei das Signal die Ausrichtungsinformationen enthält.

11. Bildgebungssystem nach einem der Ansprüche 1 bis 10, wobei der Scanmodus des Scanners (102) mindestens einen der Folgenden umfasst: B-Modus, Doppler und Farbe.

12. Verfahren zum Bedienen eines Bildgebungssystems (101) nach einem der vorherigen Ansprüche, wobei das Verfahren folgende Schritte umfasst:
a) Empfangen über den Scanner (102) einer Anfrage zum Registrieren der drahtlosen Steuervorrichtung (118) damit;
b) Übertragen durch den Scanner (102) einer Mitteilung, die angibt, dass die drahtlose Steuervorrichtung (118) damit registriert ist;
c) Empfangen an dem Scanner (102) eines Signals, das eine Position im Freiraum der registrierten Steuervorrichtung (118) angibt;
d) Positionieren durch den Scanner (102) des grafischen Cursors (122) der Anzeige (116) auf Grundlage der Position der registrierten drahtlosen Steuervorrichtung (118) im Freiraum; und
e) Auswählen eines Scanmodus des Scanners (102) über ein Bildschirmmenü.

13. Verfahren nach Anspruch 12, ferner umfassend folgende Schritte:
Erzeugen eines Mappings zwischen der Position der registrierten drahtlosen Steuervorrichtung (118) und eines Koordinatensystems der Anzeige (116); und
Positionieren des grafischen Cursors (122) der Anzeige (116) auf Grundlage des Mappings.

14. Verfahren zum Bedienen eines Bildgebungssystems (101) nach einem der Ansprüche 1 bis 11, wobei das Verfahren folgende Schritte umfasst:
a) Empfangen über den Scanner (102) einer Anfrage zum Registrieren der drahtlosen Steuervorrichtung (118) damit;
b) Übertragen durch den Scanner (102) einer Mitteilung, die angibt, dass die drahtlose Steuervorrichtung (118) damit registriert ist; und
c) Empfangen an dem Scanner (102) eines Signals von der Benutzersteuerung der Steuervorrichtung (118), die den Scanmodus davon aus der Ferne ändert.

15. Verfahren nach einem der Ansprüche 12 bis 14, ferner umfassend folgenden Schritt:
Steuern mindestens eines der folgenden Parameter: eine Verstärkung, eine Tiefe, eine Bilderfassung oder ein Zoom des Scanners (102) auf Grundlage eines Signals, das von der drahtlosen Steuervorrichtung (118) bereitgestellt wird.

## Revendications

1. Système d'imagerie (101) comprenant :
un appareil d'imagerie par ultrasons (100) comportant un scanner (102) qui inclut une interface (108) ;
une sonde de transducteur d'ultrasons (110) incluant une interface complémentaire (112) connectable à l'interface (108) du scanner (102), les interfaces (108, 112) incluant des voies et/ou des connecteurs adéquats pour transporter des signaux de commande et/ou de données entre la sonde de transducteur d'ultrasons (110) et le scanner (102), la sonde de transducteur d'ultrasons (110) comprenant un de : un transducteur à élément unique et un réseau de transducteur à éléments multiples ; et
un affichage (116) incluant un curseur graphique (122) affiché sur celui-ci ;
**caractérisé en ce que** le système d'imagerie (101) comprend en outre :
un dispositif de commande sans fil (118) incluant un composant de détection de position (202) configuré pour détecter une position du dispositif de commande sans fil (118) relative à un système de coordonnées d'espace libre et pour générer un signal indicateur de celle-ci, et un émetteur (210) configuré pour émettre le signal vers le système d'imagerie (101), le système d'imagerie (101) étant configuré pour employer le signal afin de positionner le curseur graphique (122) sur l'affichage (116) en coordination avec la position du composant de détection de position (202) dans l'espace libre, la position du curseur graphique (122) pouvant fonctionner pour sélectionner un mode de balayage du scanner (102) par l'intermédiaire d'un menu sur l'affichage ;
et **en ce que** le dispositif de commande sans fil (118) comprend en outre au moins une commande d'utilisateur (120) qui commande un paramètre du système d'imagerie (101), le paramètre pouvant fonctionner pour changer à distance le mode de balayage.

2. Système d'imagerie selon la revendication 1, dans lequel le dispositif de commande (118) comprend en outre une mémoire (206) incluant une identification unique (208), l'émetteur (210) émettant l'identification unique (208) vers le système d'imagerie (101) pour inscrire le dispositif de commande sans fil (118) de manière à faire fonctionner le système d'imagerie (101) sur la base de l'identification unique.

3. Système d'imagerie selon la revendication 2, dans lequel le dispositif de commande sans fil (118) est configuré pour faire fonctionner seulement le système d'imagerie (101) avec lequel il est inscrit.

4. Système d'imagerie selon une quelconque des revendications 1 à 3, dans lequel le dispositif de commande sans fil (118) comprend en outre :
un indicateur (200) qui fournit au moins une de : une indication visuelle, audible ou tactile en réponse à une inscription réussie entre le dispositif de commande sans fil (118) et le système d'imagerie (101).

5. Système d'imagerie selon une quelconque des revendications 1 à 4, dans lequel le paramètre correspond aussi à au moins un de : un gain, une profondeur, un zoom et une prise d'image.

6. Système d'imagerie selon la revendication 5, dans lequel au moins une commande d'utilisateur (120) est configurée pour invoquer une application qui emploie le curseur graphique (122).

7. Système d'imagerie selon une quelconque des revendications 1 à 6, dans lequel le dispositif de commande sans fil (118) est au moins un de désinfectable ou stérilisable.

8. Système d'imagerie selon une quelconque des revendications 1 à 7, dans lequel le dispositif de commande sans fil (118) est un dispositif portable manipulable à la main.

9. Système d'imagerie selon une quelconque des revendications 1 à 8, dans lequel le composant de détection de position (202) inclut un accéléromètre qui détermine une magnitude et une information de direction pour le dispositif de commande sans fil (118) dans un espace libre et le signal inclut la magnitude et l'information de direction.

10. Système d'imagerie selon une quelconque des revendications 1 à 9, dans lequel le composant de détection de position (202) inclut un gyroscope qui détermine une information d'orientation pour le dispositif de commande sans fil (118) dans un espace libre et le signal inclut l'information d'orientation.

11. Système d'imagerie selon une quelconque des revendications 1 à 10, dans lequel le mode de balayage du scanner (102) comprend au moins un de : mode B, Doppler et couleur.

12. Procédé de fonctionnement d'un système d'imagerie (101) selon une quelconque des revendications précédentes, le procédé comprenant les étapes de :
a) recevoir, via le scanner (102), une demande d'inscription du dispositif de commande sans fil (118) avec celui-ci ;
b) émettre, par le scanner (102), une notification indiquant que le dispositif de commande sans fil (118) est inscrit avec celui-ci ;
c) recevoir, au niveau du scanner (102), un signal indicateur d'une position dans l'espace libre du dispositif de commande sans fil inscrit (118) ;
d) positionner, par le scanner (102), le curseur graphique (122) de l'affichage (116) sur la base de la position du dispositif de commande sans fil (118) inscrit dans l'espace libre ; et
e) sélectionner un mode de balayage du scanner (102) via un menu sur l'affichage.

13. Procédé selon la revendication 12, comprenant en outre les étapes de :
générer une mise en correspondance entre la position du dispositif de commande sans fil inscrit (118) et un système de coordonnées de l'affichage (116) ; et
positionner le curseur graphique (122) de l'affichage (116) sur la base de la mise en correspondance.

14. Procédé de fonctionnement d'un système d'imagerie (101) selon une quelconque des revendications 1 à 11, le procédé comprenant les étapes de :
a) recevoir, via le scanner (102), une demande d'inscription du dispositif de commande sans fil (118) avec celui-ci ;
b) émettre, par le scanner (102), une notification indiquant que le dispositif de commande sans fil (118) est inscrit avec celui-ci ;
c) recevoir, au niveau du scanner (102), un signal provenant de la commande d'utilisateur du dispositif de commande (118) qui change à distance le mode de balayage de celui-ci.

15. Procédé selon une quelconque des revendications 12 à 14, comprenant en outre l'étape consistant à :
commander au moins un de : un gain, une profondeur, une prise d'images ou un zoom du scanner (102) sur la base d'un signal fourni par le dispositif de commande sans fil (118).
